# EUROPÄISCHE PATENTSCHRIFT

(11) **EP 3 242 802 B1**
(45) Veröffentlichungstag und Bekanntmachung des Hinweises auf die Patenterteilung: **19.08.2020**
(21) Anmeldenummer: 16714334.6
(22) Anmeldetag: 24.03.2016
(51) Int. Cl.: B60B 3/14, B60B 3/18, B60K 7/00, A61B 50/13, B60B 19/00, B60B 37/10

(54) **MOBILES MEDIZINISCHES GERÄT MIT MINDESTENS EINEM MOTORISCH ANGETRIEBENEN RAD**
MOBILE MEDICAL DEVICE WITH AT LEAST ONE MOTOR-DRIVEN WHEEL
APPAREIL MÉDICAL MOBILE COMPORTANT AU MOINS UNE ROUE ENTRAÎNÉE PAR UN MOTEUR

(30) Priorität: 16.04.2015 DE 102015206909
(43) Veröffentlichungstag der Anmeldung: 15.11.2017
(73) Patentinhaber: Siemens Healthcare GmbH, 91052 Erlangen (DE)
(72) Erfinder: DIRAUF, Franz, 96250 Ebensfeld (DE); DIETRICH, Florian, 91083 Baiersdorf (DE)
(86) Internationale Anmeldenummer: PCT/EP2016/056515
(87) Internationale Veröffentlichungsnummer: WO 2016/165925

(56) Entgegenhaltungen:
- WO-A1-2015/119999
- WO-A1-2015/120000
- CN-U- 201 494 299
- CN-U- 203 226 834
- DE-C1- 19 949 408
- US-A1- 2014 054 952
- US-B1- 8 549 705

## Beschreibung

Mobiles medizinisches Gerät mit mindestens einem motorisch angetriebenen Rad

### Gebiet der Erfindung

Die Erfindung betrifft ein mobiles medizinisches Gerät, das mit Hilfe von motorisch angetriebenen Rädern auf einer Bodenoberfläche verfahrbar ist. Bei dem medizinischen Gerät handelt es sich beispielsweise um eine Patientenliege oder ein C-Bogen Röntgengerät.

### Hintergrund der Erfindung

Mobile medizinische Geräte weisen in der Regel ein Fahrwerk mit manuell oder motorisch angetriebenen Rädern auf und werden in medizinischen Bereichen vor allem in Operationssälen verwendet. Die Bodenoberfläche kann in einer derartigen Umgebung beispielsweise durch Körperflüssigkeiten, Spülflüssigkeiten oder Verbrauchsmaterial verunreinigt sein.

Die Offenlegungsschrift US 2014/0379130 A1 offenbart beispielsweise die Verwendung von Mecanum-Rädern für mobile medizinische Geräte. Diese sind aufgrund ihrer guten Manövrierbarkeit besonders für die Nutzung im klinischen Umfeld geeignet.

Das Mecanum-Rad ist ein Rad, das einem damit ausgestatteten Fahrzeug omnidirektionale Fahrmanöver erlaubt, ohne mit einer mechanischen Lenkung ausgestattet sein zu müssen. Auf dem Umfang des Rades sind mehrere drehbar gelagerte tonnenförmige Rollen meist im Winkel von 45 Grad zur Achse des gesamten Rades angebracht. Ausschließlich diese Rollen stellen den Kontakt zur Bodenoberfläche her. Die Rollen haben keinen direkten eigenen Antrieb und können sich frei um ihre schräge Lagerachse drehen. Das gesamte Mecanum-Rad wird von einem Antriebsmotor mit veränderbarer Drehrichtung und variabler Drehzahl angetrieben. Um das omnidirektionale Fahrmanöver zu realisieren, werden die Drehzahl und die Drehrichtung eines jeden Rades einzeln angesteuert.

In der Gebrauchsmusterschrift CN 203226834 U wird ein mobiles C-Bogen Röntgengerät mit omnidirektionalen Rädern offenbart und das Wirkprinzip der individuellen Ansteuerung eines jeden Rades erläutert.

Motorisch angetriebene Räder und omnidirektionale Räder, wie beispielsweise die Mecanum-Räder, sind aufgrund ihrer Struktur oder ihrer Befestigung schwer zu reinigen. Es ist üblich, den Boden und die Geräte in einem Operationssaal durch Abwischen zu reinigen und diese in bestimmten Zeitabständen einer besonders gründlichen Reinigung und Desinfektion zu unterziehen. Eine gründliche Reinigung der Räder ist schwer möglich.

Um die Nutzung mobiler medizinischer Geräte, wie C-Bogen Röntgengeräte und mobile Patientenliegen, vor allem während chirurgischer Eingriffe zu erleichtern, sind Maßnahmen zur Verringerung der Kontaminationsgefahr der mobilen medizinischen Geräte zu treffen.

In der Patentanmeldung US 2014/054952 A1 ist eine schnell lösbare Naben-und-Rad-Anordnung offenbart. Die Anordnung umfasst Innen- und Außenhülsen, die um einen drehbaren Verriegelungsring angeordnet sind. Der Verriegelungsring ist in eine verriegelte Position vorspannbar und vorübergehend in eine entriegelte Position bringbar.

In der Patentschrift US 8 549 705 B1 ist ein Hohlradsatz beschrieben, der ein hohles Drehrad, ein Zahnrad, eine Begrenzungshülse sowie einen Begrenzungs- und Befestigungsring umfasst. Die Radhalterung weist einen Befestigungsarm auf, an dem eine Radgabel vorgesehen ist. Das hohle Drehrad ist auf die Radgabel aufgezogen.

Die Patentschrift DE 19949408 C1 beschreibt, dass ein Antriebsrad umfassend eine Radnabe über eine Steckachse an einem mobilen medizinischen Gerät in Form eines Rollstuhls, insbesondere an seinem Rollstuhlgestell befestigt werden kann. Dazu ist ein Mechanismus umfassend Sperrkugeln vorgesehen, die über ein Betätigungselement in Form eines manuell zu bewegenden Bügels betätigt und zwischen einer Verriegelungsposition und einer Freigabeposition verstellt werden kann. In der Verriegelungsposition des Bügels besteht eine sichere Verbindung zwischen Antriebsrad und Fahrzeuggestell. Ein Antrieb des Antriebsrades mittels Elektromotor wird verhindert, sofern die Verbindung nicht sicher ist.

### Zusammenfassung der Erfindung

Es ist Aufgabe der Erfindung, ein mobiles medizinisches Gerät mit motorisch angetriebenen Rädern anzugeben, das in einer verunreinigten Umgebung eingesetzt werden kann.

Gemäß der Erfindung wird die gestellte Aufgabe mit dem mobilen medizinischen Gerät mit mindestens einem motorisch angetriebenen Rad des unabhängigen Patentanspruchs gelöst. Vorteilhafte Weiterbildungen sind in den abhängigen Ansprüchen angegeben.

Erfindungsgemäß wird das motorisch angetriebene Rad so ausgebildet, dass es mit bloßen Händen, das heißt von Hand und ohne Hilfsmittel, wie Werkzeuge, von einer drehbaren Nabe des mobilen medizinischen Geräts abziehbar und wieder aufsteckbar ist.

Die Erfindung beansprucht ein mobiles medizinisches Gerät mit mindestens einem motorisch angetriebenen Rad, mit dem das mobile medizinische Gerät verfahrbar ist. Das motorisch angetriebene Rad ist auf einer dem motorisch angetriebenen Rad zugehörigen Nabe angeordnet und kann von Hand und ohne Hilfsmittel reversibel von der Nabe abgenommen und reversibel auf die Nabe aufgesteckt werden. Weiterhin umfasst das motorisch angetriebene Rad eine Verriegelungsvorrichtung, mit welcher das motorisch angetriebene Rad lösbar fest mit der Nabe verbindbar ist und von der Nabe gelöst werden kann. Darüber hinaus weist die Verriegelungsvorrichtung ein ausklappbares Griffelement auf, das an der dem medizinischen Gerät abgewandten Stirnfläche des motorisch angetriebenen Rads angeordnet ist.

Die Erfindung bietet den Vorteil, dass zum Reinigen des motorisch angetriebenen Rads dieses sehr einfach von dem medizinischen Gerät entfernt und beispielsweise in einer geeigneten Wasch- und Desinfektionseinrichtung gereinigt werden kann.

In einer Ausführungsform der Erfindung ist das motorisch angetriebene Rad als omnidirektionales Rad ausgebildet.

In einer Weiterbildung ist die Nabe drehbar auf einer Achse des mobilen medizinischen Gerätes gelagert.

Des Weiteren können auf dem Umfang der Nabe Nuten angeordnet sein und in diese Nuten korrespondierende Führungselemente der Verriegelungsvorrichtung eingreifen, wobei die Verriegelungsvorrichtung verdrehbar ausgebildet ist.

In einer weiteren Ausführungsform weist die verdrehbare Verriegelungsvorrichtung Nuten auf und auf dem Umfang der Nabe sind in die Nuten eingreifende korrespondierende Führungselemente angeordnet.

In einer weiteren Ausführungsform können die Verriegelungsvorrichtung und die Nabe derart ausgebildet sein, dass das Griffelement nur in einer verriegelten Position des motorisch angetriebenen Rads einklappbar ist.

In einer Weiterbildung der Erfindung weist das medizinische Gerät ein über das motorisch angetriebene Rad klappbares schutzblechartiges Abdeckelement auf. Das Abdeckelement ist nur in einer verriegelten Position des motorisch angetriebenen Rads klappbar.

In einer Weiterbildung der Erfindung weist das mobile medizinische Gerät eine Hebevorrichtung auf, die das motorisch angetriebene Rad von einer Bodenoberfläche abheben kann.

In einer weiteren Ausführungsform der Erfindung ist in der Achse ein Elektromotor angeordnet, der das motorisch angetriebene Rad antreibt.

Das mobile medizinische Gerät kann außerdem mindestens ein nicht motorisch angetriebenes weiteres Rad aufweisen, mit dem das mobile medizinische Gerät bei einem angehobenen motorisch angetriebenen Rad verfahrbar ist.

In einer weiteren Ausführungsform weist das mobile medizinische Gerät mindestens vier omnidirektionale Räder auf, wobei diese als Mecanum-Räder ausgebildet sind.

In einer Weiterbildung der Erfindung ist die Nabe kegelstumpfförmig ausgebildet und das motorisch angetriebene Rad weist eine korrespondierende kegelstumpfförmige Vertiefung auf.

In einer weiteren Ausführungsform der Erfindung ist auf dem Umfang der Nabe mindestens ein Verdrehsicherungselement angeordnet, welches das motorisch angetriebene Rad verdrehsicher auf der Nabe hält.

Weitere Besonderheiten und Vorteile der Erfindung werden aus den nachfolgenden Erläuterungen eines Ausführungsbeispiels anhand von schematischen Zeichnungen ersichtlich.

Es zeigen:
- Fig. 1:: ein mobiles medizinisches Gerät mit mindestens einem motorisch angetriebenen Rad,
- Fig. 2:: ein motorisch angetriebenes Rad im axialen Schnitt,
- Fig. 3:: ein motorisch angetriebenes Rad in einer Außenansicht,
- Fig. 4:: ein motorisch angetriebenes Rad als omnidirektionales Rad in einer Innenansicht und
- Fig. 5:: eine räumliche Ansicht einer Nabe.

### Detaillierte Beschreibung eines Ausführungsbeispiels

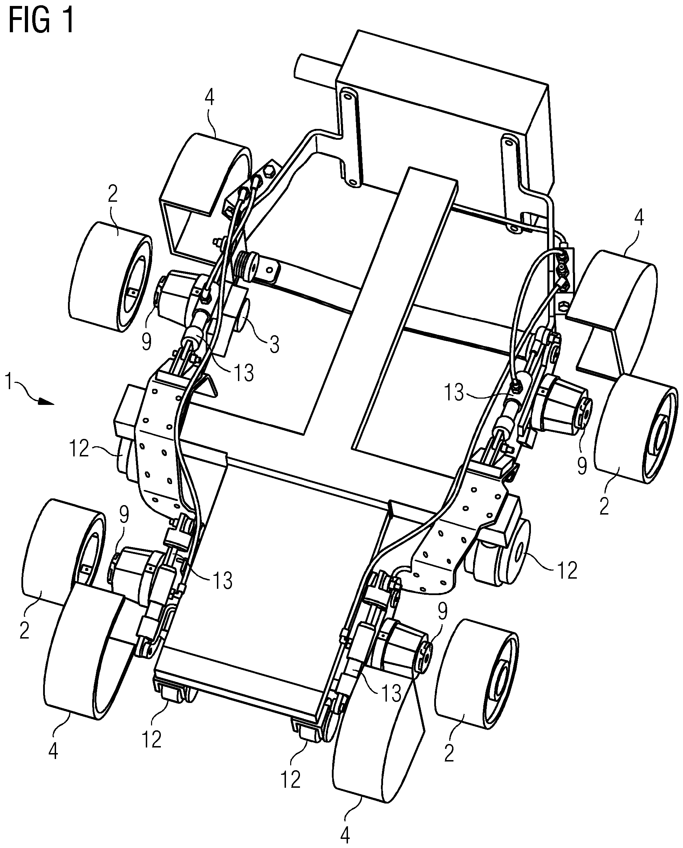

**Fig. 1** zeigt ein mobiles medizinisches Gerät 1 mit mindestens einem motorisch angetriebenen Rad 2. In diesem Ausführungsbeispiel weist das mobile medizinische Gerät 1 vier durch jeweils einen eigenen Elektromotor 3 motorisch angetriebene Räder 2 auf.

Zusätzlich ist in dieser Ausführungsform ein schutzblechartiges klappbares Abdeckelement 4 über dem motorisch angetriebenen Rad 2 angeordnet, das in geöffneter Position zu sehen ist. In geschlossener Position, das heißt in der heruntergeklappten Position, kann das Abdeckelement 4 schmutzabweisend und zusätzlich als Kabelabweiser wirken.

**Fig. 1** zeigt außerdem das mobile medizinische Gerät 1 mit einer Hebevorrichtung 13 der motorisch angetriebenen Räder 2 und mit vier weiteren Rädern 12, die nicht motorisch angetrieben sind.

Um einen Radwechsel schnell und ohne zusätzliche Hilfsmittel ausführen zu können, besitzt jedes motorisch angetriebene Rad 2 eine Hebevorrichtung 13. Damit kann jedes motorisch angetriebene Rad 2 einzeln von einer Bodenoberfläche abgehoben werden. Dabei stützt sich bei einem Radwechsel das mobile medizinische Gerät 1 auf den verbleibenden nicht angehobenen motorisch angetriebenen Rädern 2 auf dem Boden ab. Bei dieser nicht dargestellten Ausführungsform werden keine zusätzlichen Räder 12 oder beispielsweise Stützen benötigt.

In einer weiteren Ausführungsform können alle motorisch angetriebenen Räder 2 gleichzeitig von den Hebevorrichtungen 13 von der Bodenoberfläche abgehoben werden. Dabei besitzt das mobile medizinische Gerät 1 einen weiteren, zweiten Radsatz bestehend aus mindestens zwei nicht motorisch angetriebenen Rädern 12 oder Stützen, auf dem oder denen sich das mobile medizinische Gerät 1 auf der Bodenoberfläche abstützen kann. Die angehobenen motorisch angetriebenen Räder 2 stehen folglich frei und können abgenommen werden.

In einer weiteren Ausführungsform der Hebevorrichtung 13 ist diese derart ausgebildet, dass die auszuwechselnden motorisch angetriebenen Räder 2 relativ zu einem Fahrwerk des mobilen medizinischen Geräts 1 auf einer unveränderten Höhe bleiben. Ein Fachmann in dem vorliegenden Bereich der Technik versteht unter einem Fahrwerk die Gesamtheit aller Teile eines fahrbaren Gerätes, die der Verbindung des Aufbaus des fahrbaren Gerätes über die, in diesem Fall motorisch angetriebenen, Räder 2, zur Fahrbahn dienen. Dabei kann dann das gesamte Fahrwerk mit Hilfe von zusätzlichen Rädern 12 oder Stützen von der Bodenoberfläche abgehoben werden.

Vorzugsweise besitzt das mobile medizinische Gerät 1 einen zweiten Radsatz, also mindestens drei weitere, nicht motorisch angetriebene Räder 12, da diese bei einem Versagen des motorischen Antriebs durch die Elektromotoren 3 der motorisch angetriebenen Räder 2 dafür sorgen können, dass das mobile medizinische Gerät 1 trotzdem bewegt werden kann und diese somit sicherheitstechnisch relevant sind.

In einem nicht dargestellten Ausführungsbeispiel weist das mobile medizinische Gerät 1 vier motorisch angetriebene Räder 2 in Form von omnidirektionalen Rädern 15 auf, beispielsweise Mecanum-Räder. Bei Mecanum-Rädern werden die Drehrichtung und die Drehzahl jedes einzelnen Rads getrennt gesteuert. Dies bietet den Vorteil einer optimalen Manövrierbarkeit, besonders in engen Räumen, wie es in Operationssälen oft der Fall ist.

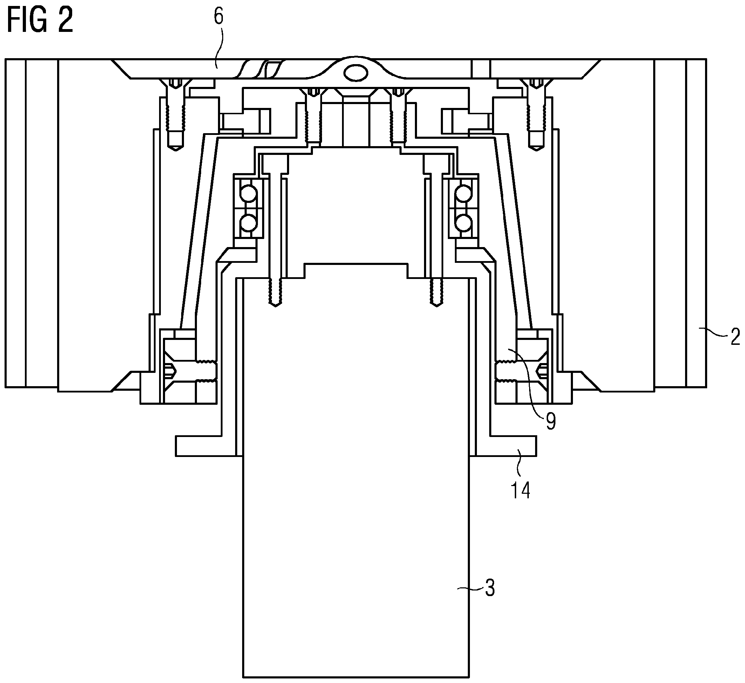

**Fig. 2** zeigt das motorisch angetriebene Rad 2, die Nabe 9, die Achse 14 und den Elektromotor 3 im axialen Schnitt. Der Elektromotor 3 befindet sich bei der dargestellten Ausführungsform in der Achse 14, die deshalb als Hohlachse oder auch als Rohr ausgeführt ist. Dabei sitzt jeweils das motorisch angetriebene Rad 2 auf einer diesem motorisch angetriebenen Rad 2 zugehörigen Nabe 9, die drehbar auf der Achse 14 gelagert ist. Dies kann durch Radlager, beispielsweise in Form von Kugellagern, ausgeführt werden. Der Elektromotor 3 treibt so über ein Getriebe und einen oder mehrere Mitnehmer, beispielsweise durch Mitnehmerstifte oder bekannte andere Mittel zur Ausführung eines Mitnehmers, die Nabe 3 an, die das Drehmoment auf das motorisch angetriebene Rad 2 überträgt.

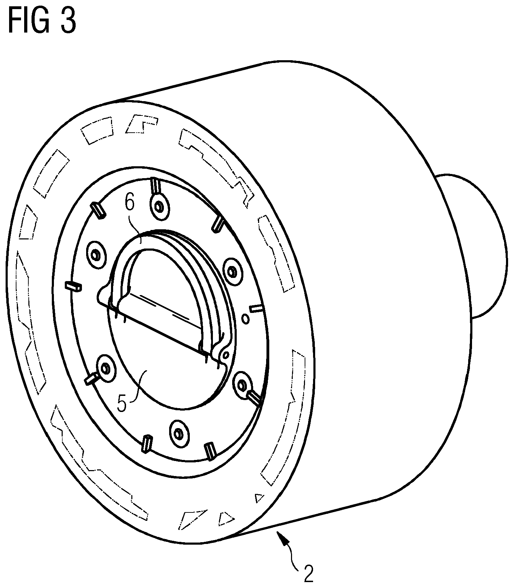

**Fig. 3** zeigt ein motorisch angetriebenes Rad 2 in einer Außenansicht. Das motorisch angetriebene Rad 2 weist eine konzentrisch in das motorisch angetriebene Rad 2 integrierte Verriegelungsvorrichtung 5 auf, wobei die Verriegelungsvorrichtung 5 ein ausklappbares Griffelement 5 auf der Stirnseite aufweist.

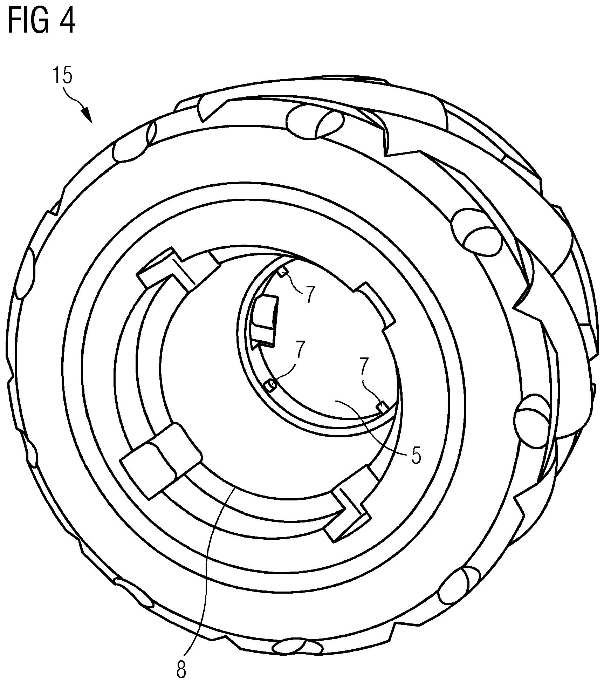

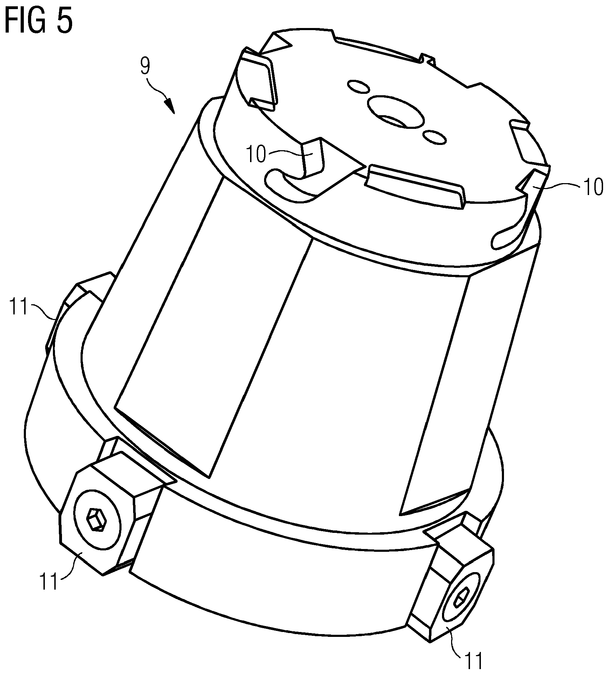

**Fig. 4** zeigt ein motorisch angetriebenes Rad 2, das als omnidirektionales Rad 15 ausgebildet ist, in einer Innenansicht bzw. von seiner Rückseite, wobei die Rückseite die sich dem mobilen medizinischen Gerät 1 zuwendende Stirnfläche des motorisch angetriebenen Rads 2 ist. Insbesondere ist das omnidirektionale Rad 15 als Mecanum-Rad ausgebildet. In dieser Ausführungsform ist im Inneren des motorisch angetriebenen Rads, also des omnidirektionalen Rads 15, beziehungsweise in seinem inneren Umfang, eine kegelstumpfförmige Vertiefung 8 ausgebildet, wobei diese kegelstumpfförmige Vertiefung 8 korrespondierend und passgenau zur kegelstumpfförmigen Ausbildung der Nabe 9 ausgebildet ist, wie in **Fig. 5** zu sehen ist und im Weiteren näher erläutert wird. Des Weiteren ist die Verriegelungsvorrichtung 5 zu sehen, die Führungselemente 6 aufweist. Die Führungselemente 6 können beispielsweise, wie in der **Fig. 4** dargestellt, als Stifte ausgebildet sein.

**Fig. 5** zeigt eine dem motorisch angetriebenen Rad 2 zugehörige Nabe 9, die auf ihrer Mantelfläche das motorisch angetriebene Rad 2 trägt und in dem vorliegenden Ausführungsbeispiel kegelstumpfförmig ausgebildet ist. Am Umfang der Nabe sind Nuten 10 und Verdrehsicherungselemente 11 ausgebildet, die dafür sorgen, dass sich das motorisch angetriebene Rad 2 sowohl im Betrieb als auch beim Verriegeln nicht gegenüber der Nabe 9 verdrehen kann.

Bezugnehmend auf die **Fig. 3****,** **Fig. 4** und **Fig. 5** wird im Weiteren das Funktionsprinzip des Abnehmens und Aufsteckens des motorisch angetriebenen Rads 2 von der Nabe 9 beschrieben. Dabei bleibt während eines gesamten Wechselvorgangs die drehbar gelagerte Nabe 9 auf der Achse 14 am Fahrwerk. Im Rahmen des Radwechsels wird also nur das motorisch angetriebene Rad 2, wie in **Fig. 4** am Beispiel des omnidirektionalen Rads 15 zu sehen, abgenommen.

Somit ist bei Bedarf eine vollständige und lückenlose Reinigung des motorisch angetriebenen Rads 2 möglich, indem dieses beispielsweise in einer im klinischen Umfeld gebräuchlichen Spül- oder Reinigungsmaschine eingelegt und gereinigt werden kann. Das motorisch angetriebene Rad 2 kann aber auch sofort durch ein bereits gereinigtes motorisch angetriebenes Rad 2 ersetzt werden.

Die Verriegelungsvorrichtung 5 dient zum sicheren Abnehmen und Befestigen des motorisch angetriebenen Rads 2, beispielsweise im Rahmen einer Reinigung des motorisch angetriebenen Rads 2.

In einer Ausführungsform der Verriegelungsvorrichtung 5 ist diese verdrehbar ausgebildet. Dabei sind am Umfang der Nabe 9 oder in der verdrehbaren Verriegelungsvorrichtung 5, beispielsweise Helix-förmige, Nuten 10 angebracht und gleichzeitig am entsprechenden Gegenstück, also entweder an der verdrehbaren Verriegelungsvorrichtung 5 oder am Umfang der Nabe 9 Führungselemente 7 angebracht. Die Führungselemente 7 bewirken bei einem Verdrehen der Verriegelungsvorrichtung 5 eine axiale Verschiebung des motorisch angetriebenen Rads 2. Somit schiebt und fixiert die Verriegelungsvorrichtung 5 bei Verdrehen das motorisch angetriebene Rad 2 auf die Nabe 9 oder löst es von der Nabe 9 bei Verdrehen in die entgegengesetzte Richtung.

In einer weiteren nicht dargestellten Ausführungsform der Verriegelungsvorrichtung 5 wird die Ver- und Entriegelung durch Verriegelungselemente realisiert, wie beispielsweise Kugeln, die in die Nuten 10, die auf dem Umfang der Nabe 9 angeordnet sind, gedrückt werden. Durch eine axial verschiebbare oder drehbare Hülse am motorisch angetriebenen Rad 2 bzw. in der Nabe 9 werden die Führungselemente 7 in einer Raststellung fixiert.

In einer weiteren nicht dargestellten Ausführungsform der Verriegelungsvorrichtung 5 wird beispielsweise mit einem zentral angeordneten klappbaren Haken, der das motorisch angetriebene Rad 2 bei Betätigung auf eine im obigen Absatz beschriebene Hülse zieht und dort fixiert, verriegelt. Der zentral angeordnete klappbare Haken kann derart ausgeführt werden, dass er durch eine entgegengesetzte Betätigung das motorisch angetriebene Rad 2 entriegelt und somit von der Nabe 9 herunter schiebt. Dabei kann zur Betätigung das Griffelement 6 beispielsweise als Handgriff ausgeführt und gleichzeitig zum Tragen des Rads ausgebildet sein. Daraus ergibt sich der Vorteil, dass das Griffelement 6 allein zum Lösen und Tragen ausreicht.

Damit bei Betätigung der Verriegelungsvorrichtung 5, wenn diese verdrehbar ausgeführt ist, sich das motorisch angetriebene Rad 2 nicht relativ zur Nabe 9 verdreht, kann die Nabe 5 auf ihrem Umfang mit dem mindestens einem Verdrehsicherungselement 11, wie in **Fig. 5** ersichtlich, ausgestattet sein. Eine Ausführungsform des Verdrehsicherungselements 11 kann durch ein auf dem Umfang der Nabe 9 herausragendes, beispielsweise ausgefrästes oder angeschraubtes, Element, das beispielsweise achteckig ausgebildet sein kann, gebildet werden.

Korrespondierend dazu sind im inneren Umfang des motorisch angetriebenen Rads 2 Nuten angebracht, in welche die Verdrehsicherungselemente 11 wirken. Sind auf dem Umfang der Nabe 9 gleichzeitig ein Verdrehsicherungselement 11 und Nuten 10 ausgebildet, können diese jeweils an den unterschiedlichen Enden der Nabe 9 angebracht sein. Beispielsweise befinden sich dann am äußeren, kleineren - falls die Nabe 9 kegelstumpfförmig ausgebildet ist, Umfang der Nabe 9, also der dem mobilen medizinischen Gerät 1 abgewandten Seite, die Nuten 10 und auf der entgegengesetzten Seite, also am äußeren, größeren Umfang - falls die Nabe 9 kegelstumpfförmig ausgebildet ist, also der dem mobilen medizinischen Gerät 1 abgewandten Seite die Verdrehsicherungselemente 11.

Das ausklappbare Griffelement 6 der Verriegelungsvorrichtung 5 dient der Betätigung der Verriegelungsvorrichtung 5. Dabei kann das ausklappbare Griffelement 6 als ausklappbarer Handgriff ausgebildet sein, sodass das entriegelte und abgenommene motorisch angetriebene Rad 2 ohne lästiges Umgreifen direkt an diesem Griffelement 6 getragen werden kann. Hieraus ergibt sich ein wesentlicher Vorteil, da das motorisch angetriebene Rad 2 mit bloßen Händen und ganz ohne zusätzliche Hilfsmittel, wie beispielsweise Werkzeuge, abgenommen werden kann.

Das ausklappbare Griffelement 6 kann so ausgeführt sein, dass dieses nur bei einem aufgesetzten motorisch angetriebenen Rad 2 in seine Ausgangstellung eingeklappt werden kann, wenn die Verriegelungsvorrichtung 5 in ihrer verriegelten Endposition steht, also bei fest montiertem motorisch angetriebenen Rad 2. Dies kann beispielsweise durch exzentrische Nocken an der Verriegelungsvorrichtung 5 und entsprechende Vertiefungen an der Nabe 9 in der Verriegelungsposition erreicht werden. Dies bringt zusätzlich einen sicherheitsrelevanten Effekt mit sich, da dadurch eine versehentlich unvollständige Verriegelung des motorisch angetriebenen Rads 2 sofort sichtbar wird.

Das Abdeckelement 4 des motorisch angetriebenen Rads 2 kann, beispielsweise bei einem Radwechsel, umgeklappt, d.h. geöffnet werden. In einer Ausführungsform ist das Zurückklappen in die Ausgangstellung, also geschlossene Stellung, des Abdeckelements 4 jedoch nur dann möglich, wenn das motorisch angetriebene Rad 2 vollständig auf die korrekte Axialposition der Nabe 9 geschoben und das Griffelement 7 der Verriegelungsvorrichtung 5 eingeklappt ist.

Obwohl die Erfindung im Detail durch die Ausführungsbeispiele näher illustriert und beschrieben wurde, ist die Erfindung durch die offenbarten Beispiele nicht eingeschränkt und andere Variationen können vom Fachmann daraus abgeleitet werden, ohne den Schutzumfang der Erfindung zu verlassen.

### Bezugszeichenliste

- 1: mobiles medizinisches Gerät
- 2: motorisch angetriebenes Rad
- 3: Elektromotor
- 4: Abdeckelement
- 5: Verriegelungsvorrichtung
- 6: Griffelement
- 7: Führungselemente
- 8: kegelstumpfförmige Vertiefung
- 9: Nabe
- 10: Nut
- 11: Verdrehsicherungselement
- 12: weiteres Rad
- 13: Hebevorrichtung
- 14: Achse
- 15: omnidirektionales Rad

## Patentansprüche

1. Mobiles medizinisches Gerät (1) mit mindestens einem motorisch angetriebenen Rad (2), mit dem das mobile medizinische Gerät (2) verfahrbar ist, wobei
- das motorisch angetriebene Rad (2) auf einer dem motorisch angetriebenen Rad (2) zugehörigen Nabe (9) angeordnet ist, **gekennzeichnet dadurch, dass**
- das motorisch angetriebene Rad (2) von Hand und ohne Hilfsmittel reversibel von der Nabe (9) abnehmbar und wieder auf die Nabe (9) steckbar ist, wobei das mobile medizinische Gerät weiter umfasst:
- eine Verriegelungsvorrichtung (5) des motorisch angetriebenen Rads (2), die ausgebildet ist, das motorisch angetriebene Rad (2) mit der Nabe (9) lösbar fest zu verbinden und von der Nabe (9) zu lösen,
- ein ausklappbares Griffelement (6) der Verriegelungsvorrichtung (5), das an der dem medizinischen Gerät (1) abgewandten Stirnfläche des motorisch angetriebenen Rads (2) angeordnet ist.

2. Mobiles medizinisches Gerät (1) nach Anspruch 1,
**dadurch gekennzeichnet,**
**dass** das motorisch angetriebene Rad (2) ein omnidirektionales Rad (15) ist.

3. Mobiles medizinisches Gerät nach Anspruch 1 oder 2, **gekennzeichnet durch:**
- eine Achse (14), auf der die Nabe (9) drehbar gelagert angeordnet ist.

4. Mobiles medizinisches Gerät nach Anspruch 1, **gekennzeichnet durch:**
- Nuten (10) auf dem Umfang der Nabe und
- in die Nuten (10) eingreifende korrespondierende Führungselemente (11) der Verriegelungsvorrichtung (5), wobei die Verriegelungsvorrichtung (5) verdrehbar ausgebildet ist.

5. Mobiles medizinisches Gerät nach Anspruch 1, **gekennzeichnet durch:**
- Nuten (10) in der Verriegelungsvorrichtung (5), wobei die Verriegelungsvorrichtung (5) verdrehbar ausgebildet ist, und
- in die Nuten (10) eingreifende korrespondierende Führungselemente (11) auf dem Umfang der Nabe (9).

6. Mobiles medizinisches Gerät nach Anspruch 1,
**dadurch gekennzeichnet,**
**dass** die Verriegelungsvorrichtung (5) und die Nabe (9) derart ausgebildet sind, dass das Griffelement (6) nur in einer verriegelten Position des motorisch angetriebenen Rads (2) einklappbar ist.

7. Mobiles medizinisches Gerät nach einem der vorhergehenden Ansprüche,
**gekennzeichnet durch:**
- ein über das motorisch angetriebene Rad (2) klappbares schutzblechartiges Abdeckelement (4), das ausgebildet ist, nur in einer verriegelten Position des motorisch angetriebenen Rads (2) über das motorisch angetriebene Rad (2) zu klappen.

8. Mobiles medizinisches Gerät nach einem der vorhergehenden Ansprüche,
**gekennzeichnet durch:**
- eine Hebevorrichtung (13), die ausgebildet ist, das motorisch angetriebene Rad (2) von einer Bodenoberfläche abzuheben.

9. Mobiles medizinisches Gerät nach einem der Ansprüche 3 bis 8,
**gekennzeichnet durch:**
- einen in der Achse (14) angeordneten Elektromotor (3), der ausgebildet ist, das motorisch angetriebene Rad (2) anzutreiben.

10. Mobiles medizinisches Gerät nach einem der vorhergehenden Ansprüche,
**gekennzeichnet durch:**
- mindestens ein nicht motorisch angetriebenes weiteres Rad (12), mit dem das mobile medizinische Gerät (1) bei einem abgehobenen motorisch angetriebenen Rad (2) verfahrbar ist.

11. Mobiles medizinisches Gerät nach Anspruch 2 bis 10, **gekennzeichnet durch:**
- mindestens vier omnidirektionale Räder (15), die als Mecanum-Räder ausgebildet sind.

12. Mobiles medizinisches Gerät nach einem der vorhergehenden Ansprüche,
**dadurch gekennzeichnet,**
**dass** die Nabe (9) kegelstumpfförmig ausgebildet ist und dass das motorisch angetriebene Rad (2) eine korrespondierende kegelstumpfförmige Vertiefung (8) aufweist.

13. Mobiles medizinisches Gerät nach einem der vorhergehenden Ansprüche,
**gekennzeichnet durch:**
- mindestens ein am Umfang der Nabe (9) angeordnetes Verdrehsicherungselement (11), das ausgebildet ist, das motorisch angetriebene Rad (2) verdrehsicher auf der Nabe (9) zu halten.

## Claims

1. Mobile medical device (1) with at least one motor-driven wheel (2) with which the mobile medical device (2) can be moved,
wherein
- the motor-driven wheel (2) is arranged on a hub (9) associated with the motor-driven wheel (2), **characterised in that**
- the motor-driven wheel (2) can be reversibly removed from the hub (9) and placed on the hub (9), by hand and without aids, wherein the mobile medical device further comprises:
- a locking device (5) of the motor-driven wheel (2) which locking device is configured to connect the motor-driven wheel (2) firmly to the hub (9) releasably and to release it from the hub (9),
- a fold-out gripping element (6) of the locking device (5) which gripping element is arranged on the end face of the motor-driven wheel (2) facing away from the medical device (1).

2. Mobile medical device (1) according to claim 1, **characterised in that**
the motor-driven wheel (2) is an omnidirectional wheel (15).

3. Mobile medical device according to claim 1 or 2, **characterised by**:
- an axle (14) on which the hub (9) is arranged rotatably mounted.

4. Mobile medical device according to claim 1,
**characterised by**:
- grooves (10) on the circumference of the hub and
- corresponding guide elements (11) of the locking device (5) which engage in the grooves (10), wherein the locking device (5) is configured so as to be rotatable.

5. Mobile medical device according to claim 1,
**characterised by**:
- grooves (10) in the locking device (5), wherein the locking device (5) is rotatably configured, and
- corresponding guide elements (11) on the circumference of the hub (9), engaging in the grooves (10).

6. Mobile medical device according to claim 1,
**characterised in that**
the locking device (5) and the hub (9) are configured such that the gripping element (6) can be folded in only in a locked position of the motor-driven wheel (2).

7. Mobile medical device according to one of the preceding claims,
**characterised by**:
- a protective sheet metal-like cover element (4) which can be folded over the motor-driven wheel (2), which is configured to fold over the motor-driven wheel (2) only in a locked position of the motor-driven wheel (2).

8. Mobile medical device according to one of the preceding claims,
**characterised by**:
- a lifting apparatus (13) which is configured to lift the motor-driven wheel (2) from a floor surface.

9. Mobile medical device according to one of claims 3 to 8, **characterised by**:
- an electric motor (3) arranged in the axle (14), said motor being configured to drive the motor-driven wheel (2) .

10. Mobile medical device according to one of the preceding claims,
**characterised by**:
- at least one non-motor-driven further wheel (12) with which the mobile medical device (1) can be moved with a raised motor-driven wheel (2).

11. Mobile medical device according to claim 2 to 10, **characterised by**:
- at least four omnidirectional wheels (15) which are configured as Mecanum wheels.

12. Mobile medical device according to one of the preceding claims,
**characterised in that**
the hub (9) is configured so as to be truncated cone-shaped and **in that** the motor-driven wheel (2) has a corresponding truncated cone-shaped depression (8).

13. Mobile medical device according to one of the preceding claims,
**characterised by**:
- at least one twist-proofing element (11) arranged on the circumference of the hub (9), configured to hold the motor-driven wheel (2) in a twist-proof manner on the hub (9) .

## Revendications

1. Appareil (1) médical mobile, comprenant au moins une roue (2) entraînée par un moteur, par laquelle (2) l'appareil médical mobile peut être déplacé,
dans lequel
- la roue (2) entraînée par un moteur est montée sur un moyeu (9), associé à la roue (2) entraînée par un moteur, **caractérisé en ce que**
- la roue (2) entraînée par un moteur peut être retirée à la main et sans moyen auxiliaire réversiblement du moyeu (9) et réenfilée sur le moyeu (9), l'appareil médical mobile comprenant en outre :
- un système (5) de verrouillage de la roue (2) entraînée par un moteur, système qui est constitué pour relier fixement de manière amovible la roue (2) entraînée par un moteur au moyeu (9) et pour la détacher du moyeu (9),
- un élément (6) de prise rabattable du système (5) de verrouillage, qui est disposé sur le côté frontal, loin de l'appareil (1) médical, de la roue (2) entraînée par un moteur.

2. Appareil (1) médical mobile suivant la revendication 1, **caractérisé en ce que** la roue (2) entraînée par un moteur est une roue (15) omnidirectionnelle.

3. Appareil (1) médical mobile suivant la revendication 1 ou 2, **caractérisé par** :
- un essieu (14), sur lequel le moyeu (9) est monté tournant.

4. Appareil (1) médical mobile suivant la revendication 1, **caractérisé par** :
- des rainures (10) sur le pourtour du moyeu et
- des éléments (11) de guidage correspondants, pénétrant dans les rainures (10), du système (5) de verrouillage, le système (5) de verrouillage étant constitué de manière à pouvoir tourner.

5. Appareil (1) médical mobile suivant la revendication 1, **caractérisé par** :
- des rainures (10) dans le système (5) de verrouillage, le système (5) de verrouillage étant constitué de manière à pouvoir tourner, et
- des éléments (11) de guidage correspondants, pénétrant dans les rainures (10), sur le pourtour du moyeu (9).

6. Appareil (1) médical mobile suivant la revendication 1, **caractérisé**
**en ce que** le système (5) de verrouillage et le moyeu (9) sont constitués de manière à ce que l'élément (6) de prise ne puisse être rabattu que dans une position verrouillée de la roue (2) entraînée par un moteur.

7. Appareil (1) médical mobile suivant l'une des revendications précédentes,
**caractérisé par** :
- un élément (4) de recouvrement du type en tôle de protection, qui peut être rabattu sur la roue (2) entraînée par un moteur et qui est constitué pour n'être rabattu sur la roue (2) entraînée par un moteur que dans une position verrouillée de la roue (2) entraînée par un moteur.

8. Appareil (1) médical mobile suivant l'une des revendications précédentes,
**caractérisé par** :
- un dispositif (13) de levage, qui est constitué pour soulever la roue (2) entraînée par un moteur d'une surface du sol.

9. Appareil (1) médical mobile suivant l'une des revendications 3 à 8,
**caractérisé par** :
- un moteur (3) électrique, qui est monté dans l'essieu (14) et qui est constitué pour entraîner la roue (2) entraînée par un moteur.

10. Appareil (1) médical mobile suivant l'une des revendications précédentes,
**caractérisé par** ;
- au moins une autre roue (12), qui n'est pas entraînée par un moteur et par laquelle l'appareil (1) médical mobile peut être déplacé lorsque la roue (2) entraînée par un moteur est soulevée.

11. Appareil (1) médical mobile suivant la revendication 2 à 10, **caractérisé par** :
- au moins quatre roues (15) omnidirectionnelles, qui sont constituées en roues mecanum.

12. Appareil (1) médical mobile suivant l'une des revendications précédentes,
**caractérisé**
**en ce que** le moyeu (9) est tronconique et **en ce que** la roue (2) entraînée par un moteur a une cavité (8) tronconique correspondante.

13. Appareil (1) médical mobile suivant l'une des revendications précédentes,
**caractérisé par** :
- au moins un élément (11) d'anti-torsion, qui est disposé sur le pourtour du moyeu (9) et qui est constitué pour maintenir la roue (2) entraînée par un moteur sur le moyeu (9) d'une manière empêchant la torsion.
